# EUROPÄISCHE PATENTSCHRIFT

(11) **EP 0 663 833 B1**
(45) Veröffentlichungstag und Bekanntmachung des Hinweises auf die Patenterteilung: **16.10.1996**
(21) Anmeldenummer: 93920795.7
(22) Anmeldetag: 22.09.1993
(51) Int. Cl.: A61K 35/64, A23L 1/076

(54) **GELEE-ROYALE**
ROYAL JELLY
GELEE ROYALE

(30) Priorität: 30.09.1992 DE 4232732
(43) Veröffentlichungstag der Anmeldung: 26.07.1995
(73) Patentinhaber: GSF - Forschungszentrum für Umwelt und Gesundheit, GmbH, D-85758 Oberschleissheim (DE)
(72) Erfinder: BENGSCH, Eberhard, F-45160 Olivet (FR)
(86) Internationale Anmeldenummer: EP9302562
(87) Internationale Veröffentlichungsnummer: WO9407512

(56) Entgegenhaltungen:
- GB-A- 838 448

## Beschreibung

Die Erfindung betrifft Standardisiertes Gelee-Royale, Verfahren zu seiner Gewinnung und seine Verwendung.

Es ist bekannt, daß die Arbeiterinnen von Apis mellifera zwischen dem 9. und dem 15. Lebenstag in den Hypopharyngaldrüsen und insbesondere in der mittleren Mandibulardrüse einen milchähnlichen eiweißreichen Futtersaft (Gelee-Royale) erzeugen. Dieser dient in jeweils geringen Mengen zur anfänglichen Fütterung der gesamten Brut in den mit Eiern bestifteten Zellen (Arbeiterinnen- und Drohnenzellen). In bedeutsamen Mengen wird er in die Zelle der allein mit der Reproduktion beschäftigten Königin eingetragen. Gelee-Royale stellt die permanente und einzige Nahrung der Bienenkönigin dar, wird von ihr ohne Ausscheidung kondensierter Produkte metabolisiert und vermittelt ihr sowohl Infektionsschutz als auch eine 50-fache Lebensdauer (bei mit der Arbeitsbiene identischem Genom) sowie die immense Reproduktionsleistung: bis zu 2 Millionen Nachkommen aus der anfänglichen Befruchtung.

Gelee-Royale kann auch bei Vertebraten, insbesondere bei Säugetieren und im Humanbereich analoge, therapeutisch interessante Wirkungen entfalten:
- Infektionsschutz vor grampositiven und gramnegativen Bakterien,
- antitumorale und antivirale Aktivitäten
weitgehend ohne zytotoxische Wirkungen auf das Wirtsgewebe.

Für die Gewinnung größerer Mengen an Gelee-Royales besteht ein erhebliches Interesse, und zwar sowohl für den kommerziellen Vertrieb der Rohsubstanzen bzw. daraus präparierter stabilisierter Dauerformen, als auch zum Zwecke der Untersuchung und Isolierung zahlreicher, bis jetzt noch weitgehend unbekannter und meist nur spurenweise vorhandener Wirkstoffe.

Es zeigt sich jedoch, daß die oben beschriebenen therapeutischen Wirkungen in ihrer Gesamtheit oder teilweise mit großer Unsicherheit belastet sind:

Insbesondere die antiviralen Wirkungen in vitro und in vivo erwiesen sich als begrenzt und nicht regelmäßig reproduzierbar, es traten sogar Schwankungen innerhalb derselben Substanzprobe auf. Die Nichtübertragbarkeit von einem Testsystem auf ein anderes schränkte die Untersuchungsmöglichkeiten ein. Bei klinischen Versuchen, insbesondere in der Tumortherapie, erwiesen sich gelegentlich erzielte spektakuläre Wirkungen als nicht wiederholbar. Die Nichtreproduzierbarkeit und Nichtübertragbarkeit führte zu einer Diskreditierung von Gelee-Royale sowohl in seiner Gesamtheit als therapeutisch aktive Rohsubstanz als auch als Forschungsgegenstand zur Suche nach neuen Wirkstoffen und deren Kommerzialisierung.

Erfindungsgemäß wurde festgestellt, daß überraschenderweise die Ursache der Aktivitätsschwankungen und -minderungen weniger in den lagerungsbedingten Zersetzungserscheinungen gewisser Wirkstoffe zu suchen ist, sondern von Anfang an durch das moderne artisanale Gewinnungsverfahren bedingt ist.

In dem vorerfindungsgemäßen Verfahren werden die Bienenstöcke in bekannter Weise mit einem Abtrenngitter für die Königin und den Weiselzellenlatten mit ca. 2 Dutzend künstlichen Weiselzellen ausgerüstet. Nach 72 h werden die Königinnenlarven aus den Weiselzellen entfernt. Die verbleibende milchige Suspension wird im Schnellverfahren durch Luftansaugen über eine verengte Röhre abgesaugt und gegen die Gefäßwand eines Auffangrezipienten geschleudert. Der Unterdruck wird von zentral gelegenen Pumpen, die gleichzeitig mehrere Arbeitsplätze bedienen, erzeugt. Sie besitzen eine beträchtliche Saugkapazität (Pumpen für Melkmaschinen). Dies führt zu einem hohen Luftdurchgang mit völliger Zerstäubung der milchigen Gelee-Royale-Masse. Pumpendruck, Extraktionsmenge (300 mg/ Königinnenzelle) und Handhabung variieren den Kontakt der Rohsubstanz mit dem Sauerstoff. Besonders ungünstig wird das Verhältnis, wenn die geringen Substanzmengen aus Arbeiterinnen- oder Drohnenzellen (10 mg) extrahiert werden.

Die Aufgabe der vorliegenden Erfindungen ist es, ein Verfahren zur Gewinnung standardisierter Gelees-Royales mit konstanter, hoher antiviraler Aktivität und optimierten übrigen Wirkungen anzugeben.

Gelöst wird diese Aufgabe durch das Kennzeichen des Patentanspruchs 2. Die Unteransprüche beschreiben vorteilhafte Ausgestaltungen des Verfahrens.

Dabei wird der Intensivkontakt der zerstäubten Gelee-Royale-Masse mit größeren Mengen Luftsauerstoff ausgeschlossen. Erfindungsgemäß werden die Extraktion und das Auffangen des Rohproduktes in einem geschlossenen System unter Argonschutzgas oder einem anderen Inertgas (z.B. Stickstoff, CO₂, Helium, Fluorchlorkohlenwasserstoffe, Krypton) realisiert.

Die Erfindung wird im folgenden anhand eines Ausführungsbeispiels näher erläutert.

In einer bevorzugten Ausführungsform der vorliegenden Erfindung werden 10-100 Weiselzellenlatten mit je ca. 20 angebrüteten Zellen in eine Schutzgasbox eingelagert. Diese ist mit einem Paar Handschuhen zur Bedienung von außen ausgerüstet und enthält eine regulierbare Ansaugpumpe, einen Auffangrezipienten, einen PTFE-schlauch und eine Ansaugröhre. Durch eine Hahnöffnung im unteren Teil des Handschuhkastens wird das Schutzgas, vorzugsweise Argon, in die Box eingeleitet, während aus einer oberen Hahnöffnung das austretende Gas über einen Schlauch in ein kleines zylindrisches Gefäß geleitet wird, auf dessen Boden sich eine brennende Kerze befindet. Das Erlöschen der Kerze zeigt die völlige Argonauffüllung an. Die Extraktion von Gelee-Royale aus den Weisel- oder sonstigen Zellen wird nun im geschlossenen System durchgeführt, das Ansauggas zirkuliert in der Box, und die extrahierende Substanz kommt nur mit dem Schutzgas in Berührung. Der zusätzliche Arbeits- und Zeitaufwand des erfindungsgemäßen Verfahrens verhält sich in angemessenen Grenzen. Zu seiner Verringerung wird weiterhin vorgeschlagen, das erfindungsgemäße Verfahren zu automatisieren und die Extraktion mikroprozessor-gesteuert ablaufen zu lassen nach einer Methode, wie sie beispielsweise bei Probenwechslern für chemische, spektrochemische und chromatographische Verfahren angewandt wird. Auch kann bei Verwendung schwerer Inertgase wie Argon, Krypton oder FCKW im offenen System verfahren werden, z.B. mit einer von oben zu öffnenden Schutzgasbox, was einen weiteren Zeitgewinn erlaubt.

Der Test der nach dem erfindungsgemäßen Verfahren gewonnenen Substanzen auf antivirale Wirkung wurde in vitro an dem System Hela-Zellen / Coxsackie-B₃-Virus realisiert. Während die unter Inertgasschutz gewonnene Gelee-Royale in 5 Tests zu jeweils einer totalen Virustiterreduktion führte, wurden in 5 Gegenversuchen nach dem alten Verfahren mit Gelee-Royale aus Weiselzellen derselben Bienenkolonie in durchschnittlich 3 Fällen nur eine Abschwächung, in 2 Fällen kein Unterschied zu nichtbehandelten Zellkultur gefunden. Die Nichtreproduzierbarkeit antiviraler Wirkung von Gelee-Royale, die nach dem alten, artisanalen Verfahren gewonnen wurde, wird durch das erfindungsgemäße Gewinnungsverfahren weitgehend behoben.

Weiterhin weist das erfindungsgemäße Produkt eine verbesserte Löslichkeit in hydrophilen Milieu auf, was auf eine raschere und gründlichere Resorbierbarkeit im Magen-Darmtrakt schließen läßt.

Klinische Versuche zeigen, daß auch in vivo die antiviralen Wirkungen (Hepatitis B HBV, Herpes HVH, Stamm 1 und 2) sowie andere therapeutische Eigenschaften der Gelee-Royale-Rohsubstanzen eine erhebliche Verbesserung erfahren und daß das erfindungsgemäße Verfahren die Erzeugung eines Spitzenproduktes mit optimalen Gesamteigenschaften erlaubt.

Demgegenüber verursacht ein zeitlich begrenzter Oberflächenkontakt der flüssigen Gelee-Royale mit Luftsauerstoff keine Aktivitätsminderung, so daß Abfüllung und Verarbeitung unter Inertgas zwar wünschenswert sind, aber ohne wesentlichen Effekt bleiben.

## Patentansprüche

1. Gelee-Royale, gewinnbar durch Absaugen aus Königinnen-, Arbeiterinnen- oder Drohnenzuchtzellen von Apis mellifera unter Inertgas.

2. Verfahren zur Extraktion von Gelee-Royale durch Absaugen aus Königinnen-, Arbeiterinnen- oder Drohnenzuchtzellen von Apis mellifera, dadurch gekennzeichnet, daß die Gewinnung unter Inertgas realisiert wird.

3. Verfahren nach Anspruch 2, dadurch gekennzeichnet, daß das Inertgas schwerer als Luft ist.

4. Verfahren nach Anspruch 2 oder 3, dadurch gekennzeichnet, daß das Inertgas Argon ist.

5. Verfahren nach einem der Ansprüche 2 bis 4, dadurch gekennzeichnet, daß die Extraktion der Gelees automatisiert wird und mikroprozessorgesteuert abläuft.

6. Gelee-Royale nach Anspruch 1 als Therapeutikum.

## Claims

1. Royal jelly, obtainable by suction from queen, worker or drone breeding cells of Apis mellifera using inert gas.

2. Method of extracting royal jelly by suction from queen, worker or drone breeding cells of Apis mellifera, characterised in that it is obtained by using inert gas.

3. Method according to claim 2, characterised in that the inert gas is heavier than air.

4. Method according to claim 2 or 3, characterised in that the inert gas is argon.

5. Method according to one of claims 2 to 4, characterised in that the extraction of the jellies is automated and occurs in a microprocessor-controlled manner.

6. Royal jelly according to claim 1 as a therapeutic agent.

## Revendications

1. Gelée royale qu'on peut obtenir par aspiration d'alvéoles de reine, d'ouvrières ou de faux-bourdons de Apis mellifera sous gaz inerte.

2. Procédé d'extraction de gelée royale par aspiration d'alvéoles de reine, d'ouvrières et de faux-bourdons de Apis mellifera,
caractérisé en ce que
l'obtention est réalisée sous gaz inerte.

3. Procédé selon la revendication 2,
caractérisé en ce que
le gaz inerte est plus lourd que l'air.

4. Procédé selon la revendication 2 ou 3,
caractérisé en ce que
le gaz inerte est de l'argon.

5. Procédé selon l'une des revendications 2 à 4,
caractérisé en ce que
l'extraction de la gelée est automatisée et est pilotée par microprocesseur.

6. Gelée royale selon la revendication 1 comme médicament.
